# EUROPEAN PATENT SPECIFICATION

(11) **EP 2 151 496 B1**
(45) Date of publication and mention of the grant of the patent: **25.04.2012**
(21) Application number: 08764247.6
(22) Date of filing: 25.04.2008
(51) Int. Cl.: C12N 15/00, C12M 1/00, C12N 15/09, C12Q 1/68

(54) **METHOD FOR EVALUATION OF DRUG SENSITIVITY BY ANALYSIS OF POMC GENE**
VERFAHREN ZUR EVALUIERUNG DER WIRKSTOFFSENSITIVITÄT MITTELS ANALYSE DES POMC-GENS
PROCEDE D'EVALUATION DE LA SENSIBILITE AUX MEDICAMENTS PAR ANALYSE D'UN GENE POMC

(30) Priority: 25.04.2007 JP 2007114968
(43) Date of publication of application: 10.02.2010
(73) Proprietor: Tokyo Metropolitan Institute of Medical Science, Tokyo 156-0057 (JP)
(72) Inventor: IKEDA, Kazutaka, Tokyo 168-0081 (JP); KASAI, Shinya, Tokyo 156-0057 (JP); HAYASHIDA, Masakazu, Tokyo 169-0073 (JP); HIGUCHI, Susumu, Fujisawa-shi Kanagawa 251-0057 (JP)
(74) Representative: Woods, Geoffrey Corlett
(86) International application number: PCT/JP2008/058083
(87) International publication number: WO 2008/133329

(56) References cited:
- WO-A2-2007/070252
- XUEI X ET AL: "Association of the kappa-opioid system with alcohol dependence" MOLECULAR PSYCHIATRY, vol. 11, no. 11, November 2006 (2006-11), pages 1016-1024, XP002598535 ISSN: 1359-4184
- MAYER P ET AL: "Allelic and somatic variations in the endogenous opioid system of humans" PHARMACOLOGY AND THERAPEUTICS, ELSEVIER, GB LNKD- DOI:10.1016/S0163-7258(01)00154-1, vol. 91, no. 3, 1 January 2001 (2001-01-01), pages 167-177, XP002990162 ISSN: 0163-7258
- XUEI XIAOLING ET AL: "The opioid system in alcohol and drug dependence: Family-based association study" AMERICAN JOURNAL OF MEDICAL GENETICS, vol. 144B, no. 7, 14 May 2007 (2007-05-14) , pages 877-884, XP002598536
- LAFORGE K.S. ET AL.: 'Opioid receptor and peptide gene polymorphisms: potential implications for addictions' EUR. J. PHARMACOL. vol. 410, 2000, pages 249 - 268, XP008123072
- CHEN Y. ET AL.: 'Pro-opiomelanocortin gene variants are associated with serum leptin and body fat in a normal femal population' EUR. J. HUM. GENET. vol. 13, 2005, pages 772 - 780, XP008123069
- SUDO Y. ET AL.: 'Association of single nucleotide polymorphisms in the promoter region of the pro-opiomelanocortin gene (POMC) with low bone mineral density in adult women' J. HUM. GENET. vol. 50, 2005, pages 235 - 240, XP019374298
- BLOMQVIST O. ET AL.: 'Pro-opiomelanocortin (POMC) gene polymorphism and association with alcohol and/or drug dependence' ALCOHOLISM CLINICAL AND EXPERIMENTAL RESEARCH vol. 24, no. 5, SUPPL., 2000, page 25A, XP008123073
- GRISEL J.E. ET AL.: 'Ethanol oral self-administration is increased in mutant mice with decreased beta-endorphin expression' BRAIN RES. vol. 835, no. 1, 1999, pages 62 - 67, XP008123071
- LEE T.H. ET AL.: 'In vivo electroporation of pro-opiomelanocortin induces analgesia in a formalin-injection pain model in rats' PAIN vol. 104, 2003, pages 159 - 167, XP008123068
- RUBINSTEIN M. ET AL.: 'Absence of opioid stress-induced analgesia in mice lacking beta-endorphin by site-directed mutagenesis' PROC. NATL. ACAD. SCI. USA vol. 93, no. 9, 1996, pages 3995 - 4000, XP000602049
- FEDER J. ET AL.: 'DNA restriction fragment analysis of the pro-opiomelanocortin gene in schizophrenia and bipolar disorders' AM. J. HUM. GENET. vol. 37, no. 2, 1985, pages 286 - 294, XP008123098
- IKEDA K. ET AL.: 'How individual sensitivity to opiates can be predicted by gene analyses' TRENDS PHARMACOL. SCI. vol. 26, no. 6, 2005, pages 311 - 317, XP004912366

## Description

### FIELD OF THE INVENTION

The present invention relates to a method for evaluating drug sensitivity by analyses of the *POMC* gene. More particularly, the present invention relates to a method for evaluating drug sensitivity by analyses of a genetic polymorphism in the 5' flanking region of the *POMC* gene.

### BACKGROUND OF THE INVENTION

Pain is a pathological condition frequently seen in various medical settings. Pain resulting from a disease or pain induced by treatment of a disease is a very serious problem for patients. Although pain sensation plays an important role as a biological warning system, undue pain could cause a significant reduction of quality of life. Recently, the importance of pain control has been recognizes, and palliative care, such as pain therapy, has made significant progress in which the opportunity to use and the amount of use of various analgesics has increased. However, because the analgesic effects and side effects of such analgesics vary greatly among individuals, predicting the analgesic effect is very important to perform an optimal administration schedule for each individual.

The proopiomelanocortin (hereinafter referred to as *POMC*) gene encodes several peptides, including β-endorphin, one of the endogenous opioid peptides. Through behavioral pharmacological studies using *POMC*-deficient mice, β-endorphin and the *POMC* gene have been found to produce analgesic effects on nociceptive behaviors induced by various stimuli and to play an important role in morphine efficacy (Cavun, S. et al., Glycyl-glutamine, an endogenous β-endorphin-derived peptide, inhibits morphine-induced conditioned place preference, tolerance, dependence, and withdrawal. J. Pharmacol. Exp. Ther. 2005, 315:949-58; Lee, T. H. et al., In vivo electroporation of proopiomelanocortin induces analgesia in a formalin-injection pain model in rats. Pain 2003, 104:159-67; Rubinstein, M. et al., Absence of opioid stress-induced analgesia in mice lacking beta-endorphin by site-directed mutagenesis. Proc. Natl. Acad. Sci. U.S.A. 1996, 93:3995-4000). Furthermore, *POMC* genetic polymorphisms and haplotypes have been reported to be associated with blood leptin concentration, body fat percentage, and bone density (Bienertova-Vasku, J. A. et al., Proopiomelanocortin gene: a new susceptibility gene for cutaneous T-cell lymphoma. Proc. Eur. Soc. Dermatol. Res. 2006, S33; Baker, M. et al., Association between common polymorphisms of the proopiomelanocortin gene and body fat distribution: a family study. Diabetes 2005, 54:2492-6; Chen, Y. et al., Proopiomelanocortin gene variants are associated with serum leptin and body fat in a normal female population. Eur. J. Hum. Genet. 2005, 13:772-80; Sudo, Y. et al., Association of single nucleotide polymorphisms in the promoter region of the pro-opiomelanocortin gene (POMC) with low bone mineral density in adult women. J. Hum. Genet. 2005, 50:235-40; Suviolahti, E. et al., Pro-opiomelanocortin gene is associated with serum leptin levels in lean but not in obese individuals. Int. J. Obes. Relat. Metab. Disord. 2003, 27:1204-11; Hixson, J. E. et al., Normal variation in leptin levels is associated with polymorphisms in the proopiomelanocortin gene, POMC. J. Clin. Endocrinol. Metab. 1999, 84:3187-91). Additionally, morphine sensitivity significantly varies among individuals, and the genetic mechanisms involved in individual differences in morphine sensitivity are becoming more clear (Ikeda, K. et al., How individual sensitivity to opiates can be predicted by gene analyses. Trends Pharmacol. Sci. 2005, 26:311-7).

### DISCLOSURE OF THE INVENTION

### Problems to be Solved by the Invention

The objective of the present invention is to provide a method for predicting drug sensitivity in each individual, specifically drug sensitivity in each individual with respect to the required number of analgesic doses, the total amount of analgesics, and vulnerability to alcohol dependence, using *POMC* genetic polymorphisms.

### Means for Solving the Invention

We focused on the *POMC* gene and have conducted extensive examinations based on conventional findings and clinical data. As a result, we identified a useful genetic polymorphism by analyzing the association between each *POMC* genetic polymorphism and sensitivity to drugs, such as analgesics and alcohol. Specifically, we found that the required number of analgesic doses and the total amount of analgesics differ between genotypes in a certain *POMC* genetic polymorphism, specifically a genetic polymorphism in the 5' flanking region of the *POMC* gene. We also found that the frequency of certain *POMC* genetic polymorphisms differs between alcoholic patients and healthy subjects, thereby accomplishing the present invention.

Thus, the present invention relates to the following:
(1) A method for evaluating drug sensitivity, comprising associating a genetic polymorphism in the 5' flanking region of *POMC* gene with an individual drug sensitivity.
In the method according to (1) above, a drug is a µ-opioid receptor-modifying agent. Examples of µ-opioid receptor-modifying agents include at least one selected from the group consisting of methamphetamine, methylenedioxymethamphetamine, amphetamine, dextroamphetamine, dopamine, cocaine, heroin, morphine, hydromorphone, oxymorphone, DAMGO, codeine, dihydrocodeine, methadone, oxycodone, hydrocodone, fentanyl, carfentanil, tramadol, meperidine, dextropropoxyphene, dextromoramide, levorphanol, etorphine, dihydroetorphine, buprenorphine, nalorphine, nalbuphine, pentazocine, levallorphan, butorphanol, naltrexone, alvimopan, naloxone, methanol, ethanol, propanol, butanol, pentanol, and diethyl ether.

In the method according to (1) above, the gene polymorphism is rs3754860 (see Table 1 below).

(2) A method for determining the type, the number of doses, and/or the dosage of a drug to be administered to an individual, comprising using the results from the evaluation by the method according to (1) above as an index.
(3) A method for predicting a side effect of a drug to be administered to an individual, comprising using the results from the evaluation by the method according to (1) above as an index.
(4) Use of an oligonucleotide comprising a nucleotide sequence of at least 10 nucleotides from SEQ ID NO: 1 or 2 including the 51st nucleotide of the nucleotide sequence represented by SEQ ID NO: 1 if the nucleotide sequence is derived from SEQ ID NO: 1 or the 51^{st} nucleotide of SEQ ID NO: 2 if the nucleotide sequence is derived form SEQ ID NO: 2, for evaluating drug sensitivity to an opioid receptor function-modifying agent. Herein, the oligonucleotide may, for example, span a length of 10-45 nucleotides.

(5) Use of an oligonucleotide comprising a nucleotide sequence represented by SEQ ID NO:1 or 2 for evaluating drug sensitivity to an opioid receptor function-modifying agent.

(6) Use according to (4) or (5) above wherein a microarray is used comprising an oligonucleotide as defined in (4) or (5) immobilized on a support.
(7) A kit for evaluating drug sensitivity to an opioid receptor function-modifying agent, comprising:
   (a) the oligonucleotide according to (4) or (5) above and/or the microarray according to (6) above; and
   (b) an opioid receptor function-modifying agent.

### EFFECT OF THE INVENTION

The present invention is able to provide a *POMC* genetic polymorphism (specifically, a genetic polymorphism in the 5' flanking region of the *POMC* gene) that allows evaluation of the difference in drug sensitivity among individuals and a method for evaluating drug sensitivity by using this genetic polymorphism. This evaluation method allows one to easily determine the optimal dosage and schedule to be prescribed for a narcotic analgesic, such as morphine, which would be extremely useful for personalized pain therapy and drug addiction treatment.

### BREIF DESCRIPTION OF THE DRAWINGS

Figure 1 is a schematic view showing the *POMC* genetic polymorphisms used for the analysis of the invention. In the figure, the black boxes represent translated regions of the gene, and the white boxes represent untranslated regions of the gene. The arrows indicate the positions of the respective polymorphisms used for the analysis.
Figure 2 is a graph showing the effects of the genotypes of a *POMC* genetic polymorphism (G-1695A) on the required number of analgesic doses during the 24 hour period following surgery (average of males and females ± standard error). In the figure, "Ave." refers to an average value of males and females, and "S.E." refers to the standard error (which likewise applies to the other figures contained herein).
Figure 3 is a graph showing the effects of the genetic alleles of *a POMC* genetic polymorphism (G-1695A) on the required number of analgesic doses during the 24 hour period following surgery (average of males and females ± standard error).

Figure 4 is a graph showing the effects of the genotypes of a *POMC* genetic polymorphism (IVS 1 + C266G) on the required number of analgesic doses during the 24 hour period following surgery (average of males and females ± standard error).
Figure 5 is a graph showing the effects of the genotypes of a *POMC* genetic polymorphism (TGA + C63T) on the required number of analgesic doses during the 24 hour period following surgery (average of males and females ± standard error).
Figure 6 is a graph showing the effects of the genotypes of a *POMC* genetic polymorphism (G-1695A) on the total amount of analgesics converted to pentazocine-equivalent dosages (average of males and females ± standard error).

Figure 7 is a graph showing the effects of the genetic alleles of a *POMC* genetic polymorphism (G-1695A) on the total amount of analgesics converted to pentazocine-equivalent dosages (average of males and females ± standard error).
Figure 8 is a graph showing the effects of the genotypes of a *POMC* genetic polymorphism (IVS1 + C266G) on the total amount of analgesics converted to pentazocine-equivalent dosages (average of males and females ± standard error).
Figure 9 is a graph showing the effects of the genotypes of a *POMC* genetic polymorphism (TGA + C63T) on the total amount of analgesics converted to pentazocine-equivalent dosages (average of males and females ± standard error).

### BEST MODES FOR CARRYING OUT THE INVENTION

1. Outline of the invention
*(1) POMC* (proopiomelanocortin)
   Narcotic analgesics such as morphine act on opioid receptors to induce analgesia. Three types of opioid receptors, mu (µ), delta (δ), and kappa (κ), are involved in analgesia. A number of endogenous opioid peptides that act on these opioid receptors are present in an organism. β-endorphin, one of these endogenous opioid peptides, provides analgesia. Although behavioral pharmacological studies using β-endorphin, as well as agonists and antagonists of respective opioid receptors, have been reported, the mechanism of action of β-endorphin on opioid receptors has not yet been confirmed.

Here, the *POMC* gene will be described. The *POMC* gene encodes an opioid peptide, β-endorphin, as well as nonopioid peptides, adrenocorticotropin (adrenocorticotropic hormone, ACTED, corticotropin-like intermediate-lobe peptide (CLIP), β- and γ-lipotropins (LPHs), and α-, β-, and γ-melanotropins (melanocyte stimulating hormones, MSHs). First, a precursor peptide is synthesized from the *POMC* gene, which is cleaved by protease at respective regions of the brain to yield respective peptides. In the anterior pituitary, ACTH, γ-MSH, β-LPH, as well as breakdown products of β-LPH (i.e., γ-LPH and β-endorpin) are yielded. β-Endorphin is further broken down into β-endorphin₁₋₂₇ which diminishes the analgesic effect of β-endorphin. In the anterior pituitary, ACTH is further broken down into α-MSH and CLIP, and γ-LPH yields β-MSH. In *POMC*-deficient mice, phenotypes such as degradation of coat/hair pigmentation, obesity, reduction of various hormones, and reduction of epinephrine production are observed, whereas a phenotype such as failure of stress-induced analgesia is observed in β-endorphin-deficient mice. In humans, POMC-deficient patients display ACTH deficiency, which eventually causes adrenal cortical hypofunction that progresses to death without ACTH administration.

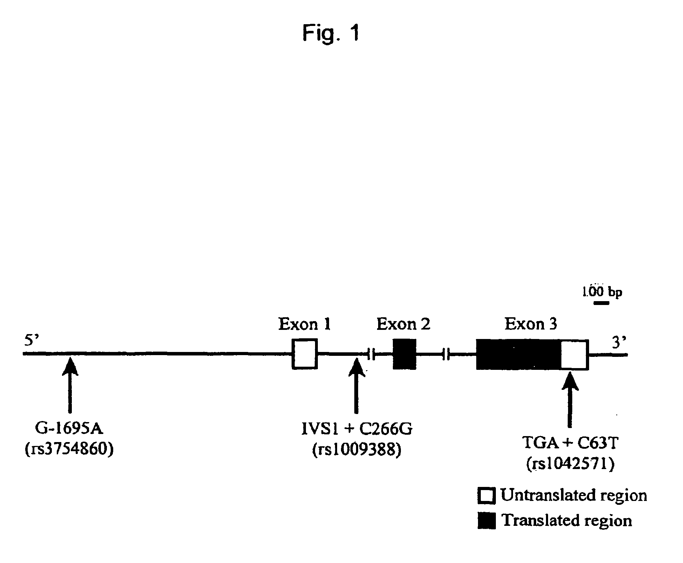

(2) *POMC* genetic polymorphisms and drug sensitivity
We analyzed genetic polymorphisms (single-nucleotide polymorphisms [SNPs], etc.) of the *POMC* gene, which have been identified to be significantly different in other diseases. The analyzed POMC genetic polymorphisms were the three polymorphisms in the 5' flanking region, intron 1, and 3' untranslated region (see Figure 1). Among these polymorphisms, one with a higher minor allele frequency was G-1695A (rs number: rs3754860) in the 5' flanking region of the *POMC* gene (see Table 1), which was subsequently analyzed.

. We determined the genotype of one of the *POMC* genetic polymorphisms (G-1695A) for patients who underwent surgery. Analyses of the associations between the genotype of the *POMC* genetic polymorphism and the required number of analgesic doses and the total amount of analgesics following surgery showed that the required number of analgesic doses and the total amount of analgesics were significantly increased for a patient group with the A/A genotype of this polymorphism compared with a patient group with the G/A genotype. Additionally, the required number of analgesic doses and the total amount of analgesics were significantly increased for a patient group without the G allele of this polymorphism compared with a patient group with the G allele. In IVS1+C266G and TGA+C63T polymorphisms, however, no significant differences were found with regard to the required number of analgesic doses and the total amount of analgesics after surgery between the patients of the respective genotypes.

Furthermore, the genotype of the *POMC* genetic polymorphism (G-1695A) was determined for alcoholic patients and healthy subjects. As a result, the G/G and G/A genotype frequencies were significantly higher for the patient group compared with the healthy subject group. A significant difference in the genotype frequency of the G-1695A polymorphism was found between alcoholic patients and healthy subjects with the 1*1 genotype of the ALDH2 gene, in which the alcoholic patients had significantly high G/G and G/A genotype frequencies. The G/G and G/A genotype frequencies of the G-1695A polymorphism were significantly higher for alcoholic patients with the 1*2 genotype of the ADH2 gene than healthy subjects with the 1*2 genotype of the ADH2 gene.

Moreover, methamphetamine-dependent patients with prolonged stimulant psychosis displayed a tendency toward higher G/G and G/A genotype frequencies for the G-1695A polymorphism compared with patients who underwent early withdrawal.
The G-1695A genetic polymorphism (rs3754860) in the 5' flanking region of the *POMC* gene appears to affect the promoter activity of the *POMC* gene, and the A/A genotype is expected to reduce the expression of the *POMC* gene.

Hence, an analysis of the genetic polymorphism of the *POMC* gene identified by the invention allows easy evaluation of differences among individuals with respect to the required number of analgesic doses, total amount of analgesics, and drug sensitivity, such as vulnerability to drug addiction. This drug sensitivity would be key information for determining the type, the number of doses and the dosages of drugs. In particular, because morphine, alcohol, and so on may cause serious social issues depending on usage and the amount used thereof, predicting the appropriate dosage for each individual before administration is very important. Moreover, because abuse of stimulant-type drugs, such as methamphetamine, is rapidly increasing and recently emerging as a serious social issue, predicting the risk of stimulant drug addiction is also important. Thus, the present invention would be quite useful for personalized pain therapy and drug addition treatment.

2. *POMC* genetic polymorphisms
A human *POMC* genetic polymorphism of the invention is the rs3754860 genetic polymorphism.

Information of the human *POMC* genetic polymorphisms identified by the invention is shown in Table 1 below. The information of the polymorphisms shown in Table 1 is associated with SNPs.
The present invention provides an oligonucleotide comprising a genetic polymorphism of the *POMC* gene shown in Table 1. A method for obtaining the information of the genetic polymorphisms of the present invention shown in Table 1 is as follows.

Genomic DNA is purified from a human blood sample by the phenol chloroform method or the like. In this regard, a commercially available genomic DNA extraction kit or device such as the GFX Genomic Blood DNA Purification Kit (GE Healthcare Japan) may be used. Next, the obtained genomic DNA is used as a template to amplify the DNA fragments in part by the polymerase chain reaction (PCR) method to obtain DNA fragments for enzymatic cleavage or sequencing. The present invention involves a polymorphism of a gene, and the DNA polymerase used for preferably the PCR method has as high fidelity as possible. According to the PCR-restriction fragment length polymorphism (PCR-RFLP) technique, primers designed based on the sequence information available from the GenBank database (hereinafter, GenBank) on the National Center for Biotechnology Information (NCBI) website (http://www.ncbi.nlm.nih.gov) are used to perform PCR amplification of about 700 nucleotides, including the polymorphisms of the *POMC* gene and the flanking sequences. These PCR fragments are cleaved with a particular restriction enzyme and subjected to electrophoresis with a visualizing dye in an agarose gel at an appropriate concentration. Subsequently, DNA is stained with ethidium bromide to identify the genotype of the genetic polymorphisms according to the lengths of the DNA fragments. In a sequencing procedure, primers whose nucleotide sequences are designed based on the sequence information available from GenBank are used for each polymorphism of the *POMC* gene to perform PCR amplification of about 700 nucleotides, including the polymorphisms and the flanking sequences. The nucleotide sequences of these PCR fragments are sequenced for about 300-700 nucleotides at a time by a sequencing procedure to obtain the genetic polymorphism of interest. As an example of the resulting polymorphism information, exemplary SNPs and their polymorphism frequencies in the *POMC* gene observed in the genomes of Japanese healthy subjects are shown in Table 1 below.

**Table 1**

| Gene Name | Region | SNP name | rs number | Submitted allele | Major allele | Minor allele | Minor allele frequencies | Sample size |
|---|---|---|---|---|---|---|---|---|
| *POMC* | 5' flanking region | G-1695A | rs3754860 | G | G | A | 0324 | 139 |
| | Intron 1 | IVS1 + C266G | rs1009388 | C | C | G | 0.032 | 139 |
| | 3' untranslated region | TGA+ C63T | rs1042571 | C | C | T | 0.011 | 139 |

In Table 1, the term "gene name" (*POMC*) refers to the human *POMC* gene.
The term "position" refers to positions on the genome of the *POMC* gene, such as the 5' flanking region, intron 1, and 3' untranslated region.
The term "SNP name" is the name of SNPs named by the present inventors after the positions on the *POMC* gene. Basically, the name includes notation of a 2-5 digit number and signs, with letters A, G, C, or T at both ends to identify the nucleotides of the SNP. For example, "G-1695A" along the row "5' flanking region" indicates that the nucleotide located at 1695 nucleotides upstream (5' direction) of the nucleotide immediately preceding exon 1 is a polymorphism of G to A substitution. Here, "G-1695A" is an SNP name given by the present inventors to the genetic polymorphism with the rs number (described later in detail) rs3754860 with reference to a contig sequence of human chromosome 2 (Accession number: NT_022184) registered with the GenBank database available on the NCBI (the National Center for Biotechnology Information) website (http://www.ncbi.nlm.nih.gov). Additionally, the polymorphism in the complementary strand corresponding to this polymorphism (G-1695A) is called by the SNP name "C-1802T" (Chen, Y. et al., Eur. J. Hum. Genet. 2005, 13:772-80; op. cit.), and thus G-1695A and C-1802T refer to substantially the same polymorphism. "C-1802T" indicates that the nucleotide located at 1802 nucleotides upstream (5' direction) of the transcription initiation site of the *POMC* gene is a polymorphism of C to T substitution. Moreover, "IVS1+C266G" along the row "Intron 1" is a polymorphism in intron 1 and indicates that the nucleotide located at 266 nucleotides downstream (3' direction) of the nucleotide at the starting point of intron 1 (IVS1) is a polymorphism of C to G substitution. Also, "TGA+C63T" along the row "3' untranslated region" indicates that the nucleotide located at 63 nucleotides downstream (3' direction) of the nucleotide following the translation stop site (TGA) is a polymorphism of C to T substitution.

The term "rs number" is a reference cluster ID number registered in the dbSNP database of genetic polymorphisms provided on the NCBI website (op. cit.). The number consists of "rs" followed by a several-digit number, and each polymorphism identified by this number is defined by species, gene name, site, and the like. In other words, each genetic polymorphism essentially has a single assigned rs number.
The term "submitted allele" refers to an allele of the sequence registered with GenBank in which a "major allele" and a "minor allele" represent major and minor alleles, respectively, among the genomes of Japanese patients who have underwent surgery. The term "minor allele frequencies" refers to a proportion of a minor allele.
The term "sample size" refers to the number of Japanese patients as test subjects who underwent surgery.

The G-1695A polymorphism shown in Table 1 is included in the *POMC* genetic polymorphisms of the invention. Furthermore, oligonucleotides comprising the G-1695A genetic polymorphism of the invention are shown in Table 2. An oligonucleotide of the invention is selected from the nucleotide sequences represented by SEQ ID NO:1 or 2, including the aforementioned polymorphisms or complementary nucleotide sequences thereto (Table 2). The IVS1 + C266G and TGA + C63T polymorphisms are included for comparative purposes only.

**Table 2**

| Gene Name | Region | SNP name | Direction of gene sequence | Sequence flanking genetic polymorphism | SEQ ID NO. |
|---|---|---|---|---|---|
| *POMC* | 5' flanking region | G-1695A | Gene strand | | 1 |
| | | | Complementary strand | | 2 |
| | Intron I | IVS1 + C266G | Gene strand | | 3 |
| | | | Complementary strand | | 4 |
| | 3' untranslated region | TGA + C63T | Gene strand | | 5 |
| | | | Complementary strand | | 6 |

Table 2 shows nucleotide sequences (SEQ ID NOS:1-6: Gene strand or complementary strand) consisting of 101 nucleotides, with a polymorphism site at the 51st nucleotide. For example, the annotations "G/A," "C/T," and "C/G" represent polymorphisms of "G" and "A," "C" and "T," and "C" and "G," respectively. Among these, the present invention provides an oligonucleotide consisting of a nucleotide sequence having at least 10 nucleotides, preferably 10-100 nucleotides, more preferably 10-45 nucleotides, still more preferably 14-25 nucleotides, including the polymorphism site (the 51^{st} nucleotide) of the nucleotide sequence shown in Table 2 (SEQ ID NOS:1-6, particularly and preferably SEQ ID NO:1 or 2) or a complementary nucleotide sequence thereto.

3. Association between *POMC* genetic polymorphisms and drug sensitivity
Polymorphisms in the *POMC* gene appear to alter the function and expression levels of β-endorphin, adrenocorticotropin (adrenocorticotropic hormone, ACTH), corticotropin-like intermediate-lobe peptide (CLIP), β- and γ-lipotropins (LPHs), α-, β-, and γ-melanotropins (melanocyte-stimulating hormones, MSHs) and the like encoded by the *POMC* gene. Therefore, *POMC* genetic polymorphisms may be associated with various phenotypes involved with β-endorphin, ACTH, CLIP, lipotropin, and MSH. Here, examples of phenotypes may include various phenotypes relative to onsets of diseases and phenotypes relative to drug sensitivity. Examples of phenotypes relative to onsets of diseases include pain sensitivity and vulnerability to drug addiction, and examples of phenotypes relative to drug sensitivity include potency of a drug, side effects of a drug, and effective duration of a drug and the like.

The association between *POMC* genetic polymorphisms and phenotypes may be examined as follows. As such, the *POMC* genetic polymorphism, a polymorphism that has significant association with other diseases (i.e., a functional polymorphism), or a tag polymorphism in the linkage block deduced from the results from linkage disequilibrium and haplotype analyses of healthy subjects is selected (for example, an SNP). Next, the frequency of this polymorphism (e.g., SNP) in test subjects (patients) is analyzed to compare with the polymorphism frequency in healthy subjects. For comparison, statistical means such as χ² test may be effectively employed.

Where the phenotype or condition is alcohol dependence, it may be classified, for example, according to the potential of accompanying nonsocial behavior, age at dependence onset, polydrug abuse, the potential for an accompanying psychosis-like state or a state of depression, the genotype of the alcohol dehydrogenase 2 (ALDH2) gene, or the genotype of the aldehyde dehydrogenase 2 (ADH2) gene. For each classification, polymorphism frequencies or genotype frequencies are compared between healthy subjects and target subjects. A polymorphism with a significant difference in the polymorphism frequency or genotype frequency from the control group may be used to evaluate susceptibility to the disease and differences in drug sensitivity. However, because the frequency of a genetic polymorphism is suggested to be influenced by ethnic background, birthplace, and the like, the aforementioned evaluation is preferably performed with a population that shows similar frequencies of the genetic polymorphisms to the population used for identifying the predicting polymorphisms (e.g., SNPs). In this regard, the application of different data from a newly analyzed population allows analysis of the different population.

4. Application of the analysis results
The results from the above analysis may be used as an index in a method for predicting sensitivity to various drugs associated with the *POMC* gene, a method for selecting treatment or prevention of a disease associated with the *POMC* gene, a method for determining an appropriate dosage of a therapeutic drug, and a method for predicting side effects and the like. An oligonucleotide or a microarray may be used to determine a genotype of an individual to allow predicting and evaluating the individual's drug sensitivity.

Examples of an opioid receptor function-modifying agent as a target for evaluating sensitivity include stimulant-type drugs, such as methamphetamine, dopamine receptor agonists, dopamine receptor antagonists, µ-, δ-, and κ-opioid receptor agonists, µ-, δ-, and κ-opioid receptor antagonists, and alcohol and the like, in which a µ-opioid receptor function-modifying agent is particularly preferable. Here, specific examples of a µ-opioid receptor-modifying agent include the following: methamphetamine, methylenedioxymethamphetamine, amphetamine, dextroamphetamine, dopamine, cocaine, heroin, morphine, hydromorphone, oxymorphone, DAMGO, codeine, dihydrocodeine, methadone, oxycodone, hydrocodone, fentanyl, carfentanil, tramadol, meperidine, dextropropoxyphene, dextromoramide, levorphanol, etorphine, dihydroetorphine, buprenorphine, nalorphine, nalbuphine, pentazocine, levallorphan, butorphanol, naltrexone, alvimopan, naloxone, methanol, ethanol, propanol, butanol, pentanol, and diethyl ether.

Moreover, the genetic polymorphisms or the method of the invention may be used to evaluate drug sensitivity or the like that differs by ethnic group. Although subjects are not particularly limited and may include Japanese, American, or European or the like, subjects in the present invention are preferably Japanese or those having similar frequencies of the genetic polymorphisms to Japanese.
Genomic samples may be extracted from blood, saliva, or skin or the like of the test subjects, which are not limited thereto as long as they allow extraction of genomic DNA. Extraction and purification methods for genomic DNA are well known. For example, genomic DNA is purified from human specimens such as blood, saliva, or skin or the like by the phenol chloroform method or the like. In this regard, a commercially available genomic DNA extraction kit or device, such as the GFX Genomic Blood DNA Purification Kit, may be used. When an SNP to be analyzed is in the exon, mRNA or total RNA may be extracted instead of genomic DNA. Hereinafter, exemplary methods for detecting a genetic polymorphism of the test sample will be described.

(1) Detection by PCR method
To amplify the test sample by PCR, a DNA polymerase with high fidelity, such as KOD Dash DNA polymerase (TOYOBO), is preferably used. The primers used are designed and synthesized such that the genetic polymorphism is included at any position of the primers that allows amplification of the target SNP in the test sample.
At the end of the amplification reaction, the amplified product is detected to determine the genotype of the genetic polymorphism.

(2) Detection by PCR-RFLP method
A DNA polymerase with high fidelity, such as KOD Dash DNA polymerase (TOYOBO), is used to amplify the test sample by PCR. The primers used are designed and synthesized such that the target SNP in the test sample is included at any position of the amplified PCR fragments. These PCR fragments are cleaved with particular restriction enzymes and subjected to electrophoresis with a visualizing dye in an agarose gel at an appropriate concentration. Finally, DNA is stained with ethidium bromide to determine the genotype of the genetic polymorphism according to the lengths of the DNA fragments.

(3) Detection by sequencing procedure
According to the present invention, a polymorphism may also be detected by a sequencing procedure based on a dideoxy procedure. A commercially available ABI series (Applied Biosystems) is employed as a sequencer used for nucleotide sequences.

(4) Detection by DNA microarray
A DNA microarray has nucleotide probes immobilized on a support, examples being a DNA chip, a gene chip, a microchip, and a bead array and the like.
First, the polynucleotide of the test sample is isolated, amplified by PCR, and labeled with fluorescent reporter molecules. Subsequently, the labeled DNA, mRNA, or total RNA are incubated with the array. Next, the hybridization pattern of this array is detected with a scanner. As hybridization data, fluorescent intensities from the fluorescent reporter molecules bound to the probe array (i.e., incorporated into the target sequence) are used. A region immobilized with a probe completely matching the target sequence gives a stronger signal than that from a region with a probe that does not match the target sequence. Because the sequence and position of each probe on the array are known, the sequence of the target polynucleotide that reacted with the probe array via complementarity can be determined.

(5) Detection by TaqMan PCR method
The TaqMan PCR method employs an allele-specific oligonucleotide labeled with a fluorescent molecule and PCR reaction using Taq DNA polymerase. The allele-specific oligonucleotide used with the TaqMan PCR method (referred to as a TaqMan probe) may be designed based on the aforementioned genetic polymorphism information.

(6) Detection of genetic polymorphism by invader assay
The invader assay is a method for detecting a genetic polymorphism by hybridizing an allele-specific oligonucleotide and a template. Use of a commercially available kit allows easy detection of the genetic polymorphism by invader assay.

5. Kit
According to the present invention, an oligonucleotide containing a POMC genetic polymorphism (e.g., SNP) site may be contained in a kit for detecting a genetic polymorphism.
The genetic polymorphism detection kit of the invention comprises one or more components required for carrying out the present invention. For example, the kit of the invention comprises a component for storing or supplying an enzyme and/or a reaction component required for carrying out genetic polymorphism detection. Examples of such components include, but are not limited to, an oligonucleotide of the invention, an enzyme buffer, dNTP, a control reagent (e.g., a tissue sample, target oligonucleotides as positive and negative controls, etc.), a labeling and/or detecting reagent, a solid support, and instructions. Alternatively, the kit of the invention may be a partial kit that contains only a part of the required components, in which case the user may prepare other components.
The kit of the invention may be provided as a microarray having the oligonucleotides immobilized on a support. The microarray has the oligonucleotide immobilized on a support, including a DNA chip, a gene chip, a microchip, and a bead array and the like.

The kit of the invention comprises an oligonucleotide comprising a *POMC* genetic polymorphism found by the invention. Thus, the *POMC* gene is isolated by a PCR method or the like by collecting blood prior to the use of a drug by a patient (e.g., before surgery or upon incidence of cancer pain) and then this gene or a fragment thereof is allowed to react with the oligonucleotide of the kit, thereby determining the genotype. The administration schedule, such as the type and dosage of drug, will be made according to the determined genotype of the genetic polymorphism. As a result, a drug effect suitable for each individual can be obtained, which is beneficial for customized medicine. For example, where morphine is used, an analgesic effect that suits each individual may be obtained while keeping side effects to a minimum.

Hereinafter, the present invention will be described in a more specific manner, although the present invention is not limited thereto.

### [EXAMPLE 1]

<Association between *POMC* genetic polymorphisms and required number of analgesic doses>
Genomic DNA was extracted from blood or mucosa of the oral cavity of 139 patients who underwent surgery to determine the genotype of genetic polymorphisms (G-1695A polymorphism) of the *POMC* gene by the PCR-RFLP method with the primer sets 1-3 shown in Table 3 below (SEQ ID NOS:7-12). The primers shown in Table 4 below (SEQ ID NOS:13-16) were used to determine the genotypes of other genetic polymorphisms (IVS1+C266G and TGA+C63T polymorphisms) of the *POMC* gene by a sequencing procedure. The data for IVS1 + C266G and TGA + C63T are included for comparative purposes only. Associations between the results of determination of the genotypes of these genetic polymorphisms and the required number of analgesic doses after surgery were analyzed.

**Table 3**

| Primer sets for determining genotype *of POMC* G-1695A polymorphism by PCR-RFLP method, lengths of amplified products, and lengths of fragments cleaved by restriction enzyme | | | | | | |
|---|---|---|---|---|---|---|
| Primer sets | Direction | Primer sequence (length) | SEQ ID NO | Length of amplified product | Restriction Enzyme | Length of fragment cleaved by restriction enzyme |
| 1 | Forward primer | 5'-CCAGTGATTCTAAATGCAGTT-3' (21 mer) | 7 | 517 bp | Rsa 1 | 240 bp/ 277 bp (G) |
| | Reverse Primer | 5'-GTAACTTCAGGAGGGTGCTGG-3' (21 mer) | 8 | | | Total 517 bp (A) |
| 2 | Forward primer | 5'-CAGTGATTGTAAATGCAGTT-3' (21 mer) | 9 | 388 bp | Rsa 1 | 111 bp/ 277 bp (G) |
| | Reverse primer | 5'-GAATTACCCTCTCACrCTGTTGC-3' (23 mer) | 10 | | | Total 388 bp (A) |
| 3 | Forward primer | 5'-CCAGTGATTCTAAATGCAGTT-3' (21 mer) | 11 | 558 bp | Rsa 1 | 277 bp/ 281 bp (G) |
| | Reverse primer | 5'-GAGATGGTGGTTTTGCCTTATTT-3' (24 mer) | 12 | | | Total 558 bp (A) |

**Table 4**

| Primer sets for determining genotypes *of POMC* IVS1+C266G and TGA+C63T polymorphisms by sequencing procedure and lengths of amplified products | | | | |
|---|---|---|---|---|
| Genetic polymorphism | Direction | Primer sequence (length) | SEQ ID NO | Length of amplified product |
| IVS1 + C266G | Forward primer | 3'-AGCCTCCCGAGACAGGTAAG-3' (20 mer) | 13 | 578 bp |
| | Reverse primer | 5'-CCTGTTGTCGGGAGTTTGAG-3' (20 mer) | 14 | |
| TGA + C63T | Forward primer | 5'-CCCAAGGACAAGCGCTAC-3' (18 mer) | 15 | 346 bp |
| | Reverse primer | 5'-CCATGCTGCTGTTATTTGAC-3' (20 mer) | 16 | |

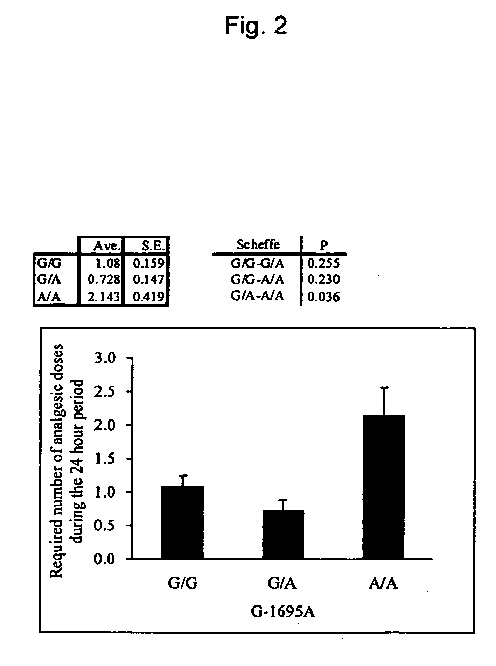

As a result, as shown in Table 5 below and Figure 2, the patient group with the A/A genotype of the G-1695A polymorphism required a significantly greater number of analgesic doses during the 24 hour period following surgery compared with the patient group with G/A (P = 0.036). In particular, females (F) with the A/A genotype tended to require a greater number of analgesic doses compared with males (M).

**Table 5**

| Effects *of POMC* genetic polymorphisms on the number of analgesic doses during the 24 hour period following surgery for patients receiving analgesics upon surgery (descriptive statistics for each genotype and sex) | | | | | |
|---|---|---|---|---|---|
| *POMC* genetic polymorphism | Genotype | Sex | Average* | Standard deviation | Number of test subjects |
| G-1695A | | F | 1.039 | 1.311 | 26 |
| | G/G | M | 1.121 | 1.111 | 33 |
| | | Sum | 1.085 | 1.193 | 59 |
| | | F | 0.724 | 1.066 | 29 |
| | G/A | M | 0.732 | 0.895 | 41 |
| | | Sum | 0.729 | 0.962 | 70 |
| | | F | 3.000 | 4.359 | 3 |
| | A/A | M | 1.286 | 1.380 | 7 |
| | | Sum | 1.800 | 2.486 | 10 |
| IVS + C266G | | F | 1.018 | 1.521 | 55 |
| | C/C | M | 0.921 | 1.055 | 76 |
| | | Sum | 0.962 | 1.267 | 131 |
| | | F | 0333 | 0.577 | 3 |
| | C/G | M | 1.250 | 0.957 | 4 |
| | | Sum | 0.857 | 0.900 | 7 |
| | | M | 1.000 | - | 1 |
| | G/G | Sum | 1.000 | - | 1 |
| TGA + C63T | | F | 1.000 | 1.500 | 57 |
| | C/C | M | 0.924 | 1.047 | 79 |
| | | Sum | 0.956 | 1.252 | 136 |
| | | F | 0.000 | - | 1 |
| | C/T | M | 1.500 | 0.707 | 2 |
| | | Sum | 1.000 | 1.000 | 3 |
| Sum | | F | 0.983 | 1.493 | 58 |
| | | M | 0.93 | 1.041 | 81 |
| | | Sum | 0.957 | 1245 | 139 |

| | | | | | |
|---|---|---|---|---|---|
| *Required number of analgesic doses during the 24 hour period following surgery. | | | | | |

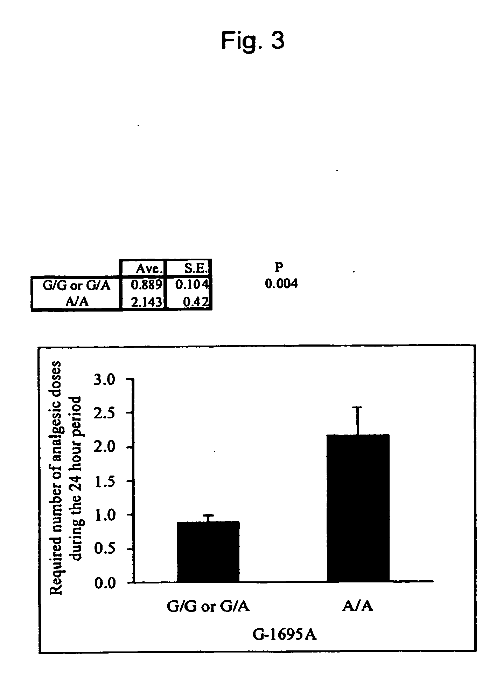

As shown in Table 6 below and Figure 3, the patient group without the G allele of the G-1695A polymorphism required a significantly greater number of analgesic doses during the 24 hour period following surgery compared with the patient group with the G allele (P = 0.004).

**Table 6**

| Effect *of POMC* G-1695A polymorphism on the number of analgesic doses during the 24 hour period following surgery for patients receiving analgesics upon surgery (descriptive statistics for each genotype and sex) | | | | |
|---|---|---|---|---|
| *POMC* G-1695A | Sex | Average* | Standard deviation | Number of test subjects |
| | F | 0.872 | 1.187 | 55 |
| G/G or G/A | M | 0.905 | 1.001 | 74 |
| | Sum | 0.892 | 1.084 | 129 |
| | F | 3.000 | 4.359 | 3 |
| A/A | M | 1.286 | 1.380 | 7 |
| | Sum | 1.800 | 2.486 | 10 |
| | F | 0.983 | 1.493 | 58 |
| Sum | M | 0.938 | 1.041 | 81 |
| | Sum | 0.957 | 1245 | 139 |

| | | | | |
|---|---|---|---|---|
| *Required number of analgesic doses during the 24 hour period following surgery. | | | | |

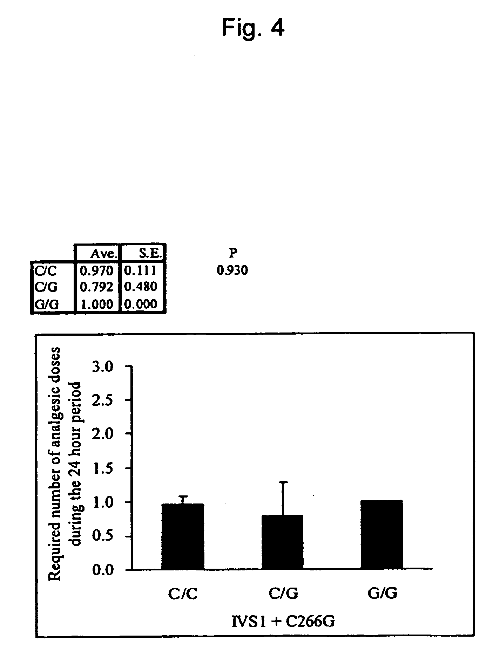

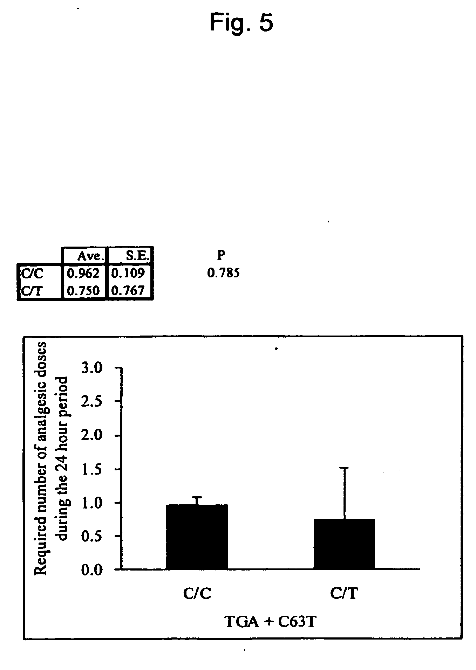

However, as shown in Table 5 above and Figure 4, no significant difference was observed in the required number of analgesic doses during the 24 hour period following surgery among the patients with the C/C, C/G, and G/G genotypes of the IVS1+C266G polymorphism (P = 0.930).
Additionally, as shown in Table 5 above and Figure 5, no significant difference was observed in the required number of analgesic doses during the 24 hour period following surgery among patients with the C/C and C/T genotypes of the TGA+C63T polymorphism (P = 0.785).
From these results, the number of analgesic doses required was proven to be readily predicted by determining the genotype of the *POMC* genetic polymorphism G-1695A.

### [EXAMPLE 2]

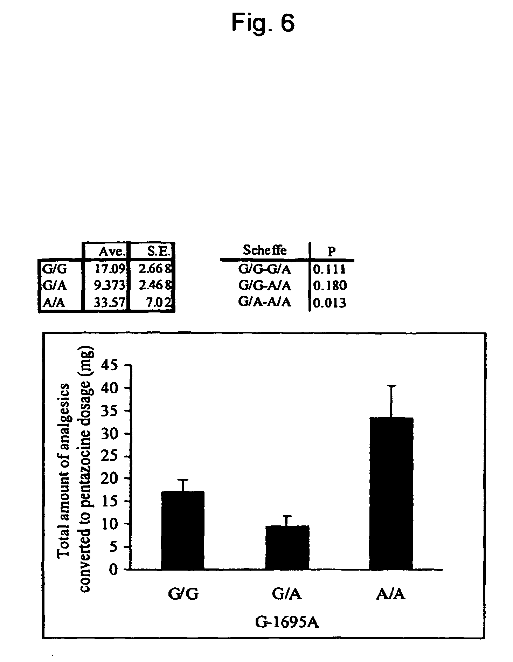

<Association between *POMC* genetic polymorphisms and total amount of analgesics>
Genomic DNA was extracted from blood or mucosa of the oral cavity of 139 patients who underwent surgery to determine the genotype of a genetic polymorphism (G-1695A) of the *POMC* gene by the PCR-RFLP method with the primer sets 1-3 shown in Table 3 of EXAMPLE 1 (SEQ ID NOS:7-12). Additionally, the primers shown in Table 4 of EXAMPLE 1 (SEQ ID NOS:13-16) were used to determine the genotypes of IVS1+C266G and TGA+C63T polymorphisms of the *POMC* gene by a sequencing procedure. Associations between the results of determining the genotypes of these genetic polymorphisms and the total amount of analgesics after surgery were analyzed. Data for the IVS1 + C66G and TGA + C63T polymorphisms are included for comparative purposes only.
As a result, as shown in Table 7 below and Figure 6, the patient group with the A/A genotype of the G-1695A polymorphism had a significantly greater total amount of analgesics converted to pentazocine-equivalent dosages compared with the patient group with G/A (P = 0.013). In particular, females with the A/A genotype tended to have a greater total amount of analgesics converted to pentazocine-equivalent dosages. In this regard, the phrase "total amount of analgesics converted to pentazocine-equivalent dosages" refers to a total amount of analgesics (milligrams) in which the amount of each of the administered analgesics is converted to an equivalent amount of pentazocine.

**Table 7**

| Effects of *POMC* genetic polymorphisms on total amount of analgesics converted to pentazocine-equivalent dosages for patients receiving analgesics upon surgery (descriptive statistics for each genotype and sex) | | | | | |
|---|---|---|---|---|---|
| *POMC* genetic polymorphism | Genotype | Sex | Average* | Standard deviation | Number of test subjects |
| G-1695A | | F | 17.596 | 27.978 | 26 |
| | G/G | M | 16.591 | 18.633 | 33 |
| | | Sum | 17.034 | 23.004 | 59 |
| | | F | 9.052 | 13.942 | 29 |
| | G/A | M | 9.695 | 13.546 | 41 |
| | | Sum | 9.429 | 13.615 | 70 |
| | | F | 42.500 | 61.084 | 3 |
| | A/A | M | 24.643 | 26.237 | 7 |
| | | Sum | 30.000 | 36.912 | 10 |
| IVS + C266G | | F | 15.272 | 25.616 | 55 |
| | C/C | M | 13.322 | 17.129 | 76 |
| | | Sum | 14.141 | 21.042 | 131 |
| | | F | 2.500 | 4.330 | 3 |
| | C/G | M | 22.500 | 25.981 | 4 |
| | | Sum | 13.929 | 21.402 | 7 |
| | | M | 15.000 | - | 1 |
| | G/G | Sum | 15.000 | - | 1 |
| TGA + C63T | | F | 14.868 | 25.255 | 57 |
| | C/C | M | 13.196 | 16.866 | 79 |
| | | Sum | 13.897 | 20.727 | 136 |
| | | F | 0.000 | - | 1 |
| | C/T | M | 37.500 | 31.820 | 2 |
| | | Sum | 25.000 | 31.225 | 3 |
| Sum | | F | 14.612 | 25.109 | 58 |
| | | M | 13.796 | 17.447 | 81 |
| | | Sum | 14.137 | 20.905 | 139 |

| | | | | | |
|---|---|---|---|---|---|
| *Total amount of analgesics (milligrams) converted to pentazocine-equivalent dosages. | | | | | |

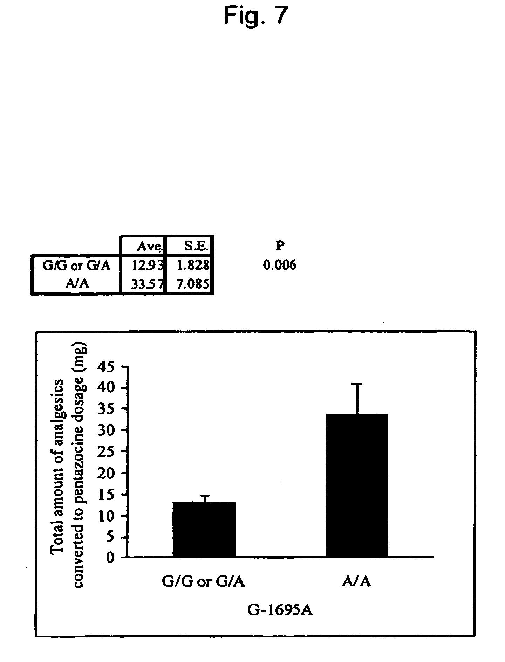

Moreover, as shown in Table 8 below and Figure 7, the patient group without the G allele of the G-1695A polymorphism had a significantly greater total amount of analgesics converted to pentazocine-equivalent dosages compared with the patient group with the G allele (P = 0.006).

**Table 8**

| Effect of *POMC* G-1695A polymorphism on total amount of analgesics converted to pentazocine-equivalent dosages for patients receiving analgesics upon surgery (descriptive statistics for each genotype and sex) | | | | |
|---|---|---|---|---|
| *POMC* G-1695A | Sex | Average* | Standard deviation | Number of test subjects |
| | F | 13.091 | 21.948 | 55 |
| G/G or G/A | M | 12.770 | 16.268 | 74 |
| | Sum | 12.907 | 18.820 | 129 |
| | F | 42.500 | 61.084 | 3 |
| A/A | M | 24.643 | 26.237 | 7 |
| | Sum | 30.000 | 36.912 | 10 |
| | F | 14.612 | 25.109 | 58 |
| Sum | M | 13.796 | 17.447 | 81 |
| | Sum | 14.137 | 20.905 | 139 |

| | | | | |
|---|---|---|---|---|
| *Total amount of analgesics (milligrams) converted to pentazocine-equivalent dosages. | | | | |

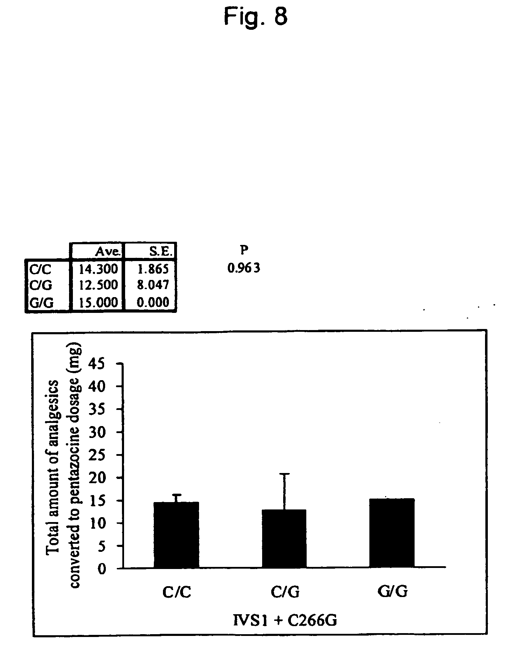

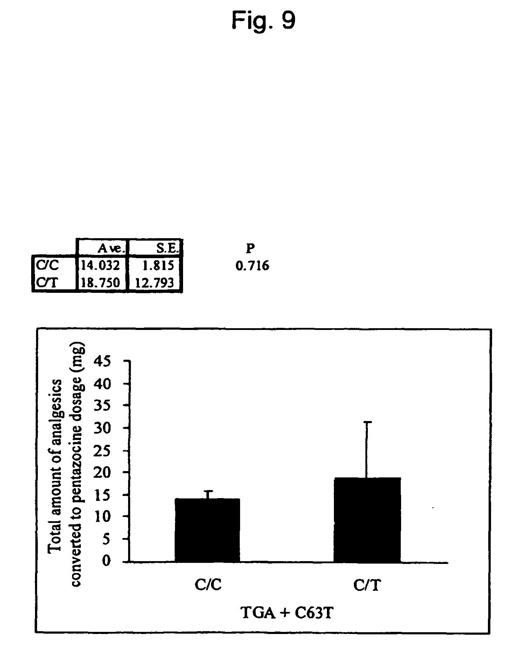

However, as shown in Table 7 above and Figure 8, no significant difference was observed in the total amount of analgesics converted to pentazocine-equivalent dosages among the patients with the C/C, C/G, and G/G genotypes of the NS1+C266G polymorphism (P = 0.963).
Additionally, as shown in Table 7 above and Figure 9, no significant difference was observed in the total amount of analgesics converted to pentazocine-equivalent dosages among the patients with the C/C and C/T genotypes of the TGA+C63T polymorphism (*P* = 0.716).
From these results, the total amount of analgesics was proven to be readily predicted by determining the genotype of the *POMC* genetic polymorphism G-1695A.

### [EXAMPLE 3]

<Association between *POMC* genetic polymorphism (G-1695A) and alcohol sensitivity>
Genomic DNA was extracted from blood of 425 alcoholic patients and 339 healthy subjects to determine the genotype of a genetic polymorphism (G-1695A) of the *POMC* gene by the PCR-RFLP method with the primer sets 1-3 shown in Table 3 of EXAMPLE 1 (SEQ 1D NOS:7-12).
As a result, as shown in Table 9 below, the frequency of each of the G/G, G/A and A/A genotypes tended to differ between the alcoholic patients and the healthy subjects (P = 0.072). Furthermore, as shown in Table 9 below, the G/G and G/A genotype frequencies were found to be significantly high in the alcoholic patients (P = 0.024).

**Table 9**

| Comparison of genotype frequencies of *POMC* G-1695A polymorphism between healthy subjects and alcoholic patients | | | | | |
|---|---|---|---|---|---|
| SNP name | *POMC* G-1695A | | | | |
| (sample number) | Genotype frequency (%) | | | | |
| | G/G | G/A | A/A | G/G or G/A | A/A |
| Healthy subjects (339) | 178 | 122 | 39 | 300 | 39 |
| | (52.5%) | (36.0%) | (11.5%) | (88.5%) | (11.5%) |
| Alcoholic patients (425) | 229 | 167 | 29 | 396 | 29 |
| | (53.9%) | (39.3%) | (6.8%) | (93.2%) | (6.8%) |
| | | | χ² = 5.254 | | χ² =5.096 |
| | | | P = 0.0723 | | *P* = 0.0240 |

### [EXAMPLE 4]

<Association between the *POMC* genetic polymorphism (G-1695A) classified by *ALDH2* genotype and alcohol sensitivity>
Genomic DNA was extracted from blood of 425 alcoholic patients and 339 healthy subjects to determine the genotype of a genetic polymorphism (G-1695A) of the *POMC* gene by the PCR-RFLP method with the primer sets 1-3 shown in Table 3 of EXAMPLE 1 (SEQ ID NOS:7-12).
As a result, as shown in Table 10 below, a significant difference was observed in the genotype frequencies between the alcoholic patients and the healthy subjects with the 1*1 genotype of the *ALDH2* genotype (P = 0.026). Moreover, as shown in Table 10 below, the G/G and G/A genotype frequencies were found to be significantly high in the alcoholic patients (P = 0.012).

**Table 10**

| Comparison of genotype frequencies of *POMC* G-1695A polymorphism between healthy subjects and alcoholic patients classified by *ALDH2* genotype | | | | | | |
|---|---|---|---|---|---|---|
| SNP name | | *POMC* G-1695A | | | | |
| (sample number) | *ALDH2* | Genotype Frequency (%) | | | | |
| | genotype | G/G | G/A | A/A | G/G or G/A | A/A |
| | (sample number) | | | | | |
| | 1*1 | 103 | 64 | 27 | 167 | 27 |
| | (194) | (53.1%) | (33.0%) | (13.9%) | (86.1%) | (13.9%) |
| Healthy | 1*2 | 68 | 50 | 9 | 118 | 9 |
| subjects (339) | (127) | (53.5%) | (39.4%) | (7.1%) | (92.9%) | (7.1%) |
| | 2*2 | 7 | 8 | 3 | 15 | 3 |
| | (18) | (38.9%) | (44.4%) | (16.7%) | (833%) | (16.7%) |
| | | | | | | |
| Alcoholic | 1*1 | 201 | 151 | 28 | 352 | 28 |
| patients (425) | (380) | (52.9%) | (39.7%) | (7.4%) | (92.6%) | (7.4%) |
| | 1*2 | 28 | 16 | 1 | 44 | 1 |
| | (45) | (62.2%) | (35.6%) | (2.2%) | (97.8%) | (2.2%) |

### [EXAMPLE 5]

<Association between *POMC* genetic polymorphism (G-1695A) classified by *ADH2* genotype and alcohol sensitivity>
Genomic DNA was extracted from blood of 425 alcoholic patients and 339 healthy subjects to determine the genotype of a genetic polymorphism (G-1695A) of the *POMC* gene by the PCR-RFLP method with the primer sets 1-3 shown in Table 3 of EXAMPLE 1 (SEQ ID NOS:7-12).
As a result of comparisons between the alcoholic patients and the healthy subjects with the 1*2 genotype of *ADH2* genotype, G/G and G/A genotype frequencies were found to be significantly high in the alcoholic patients (P = 0.046) as shown in Table 11 below.

**Table 11**

| Comparison of genotype frequencies of *POMC* G-1695A polymorphism between healthy subjects and alcoholic patients classified by *ADH2* genotype | | | | | | |
|---|---|---|---|---|---|---|
| SNP name | | *POMC* G-1695A | | | | |
| (sample number) | *ADH2* | Genotype frequency (%) | | | | |
| | genotype | G/G | G/A | A/A | G/G or G/A | A/A |
| | (sample number) | | | | | |
| | 1*1 | 13 | 7 | 1 | 20 | 1 |
| | (21) | (61.9%) | (333%) | (4.8%) | (95.2%) | (4.8%) |
| Healthy | | | | | | |
| subjects | 1*2 | 67 | 45 | 17 | 112 | 17 |
| (339) | (129) | (51.9%) | (34.9%) | (132%) | (86.8%) | (13.2%) |
| | | | | | | |
| | 2*2 | 98 | 70 | 21 | 168 | 21 |
| | (189) | (51.9%) | (37.0%) | (11.1%) | (88.9%) | (11.1%) |
| | | | | | | |
| | | | | | | |
| | 1*1 | 58 | 58 | 7 | 116 | 7 |
| Alcoholic | (123) | (472%) | (472%) | (5.7%) | (94.3%) | (5.7%) |
| patients | | | | | | |
| (425) | 1*2 | 81 | 55 | 9 | 136 | 9 |
| | (145) | (55.9%) | (37.9%) | (62%) | (93.8%) | (6.2%) |
| | | | | | | |
| | 2*2 | 90 | 54 | 13 | 144 | 13 |
| | (157) | (57.3%) | (34.4%) | (8.3%) | (91.7%) | (8.3%) |

### [EXAMPLE 6]

<Association between *POMC* genetic polymorphism (G-1695A) and methamphetamine sensitivity>
Genomic DNA was extracted from blood of 164 stimulant-type drug (methamphetamine)-dependent patients to determine the genotype of a genetic polymorphism (G-1695A) of the *POMC* gene by the PCR-RFLP method with the primer sets 1-3 shown in Table 3 of EXAMPLE 1 (SEQ ID NOS:7-12).
As a result, as shown in Table 12 below, when the patient group who underwent early withdrawal and experienced stimulant psychosis (less than 1 month) were compared with the patient group with prolonged stimulant psychosis (1 month or longer), G/G and G/A genotype frequencies were found to be significantly high in the patient group with prolonged delusion and hallucination (P = 0.072).

**Table 12**

| Comparison of genotypes *of POMC* G-1695A polymorphism in methamphetamine- dependent patients classified according to duration of delusions and hallucinations | | | | | |
|---|---|---|---|---|---|
| SNP name | *POMC* G-1695A | | | | |
| Methamphetamine-dependent patients | Genotype frequency (%) | | | | |
| (sample number) | G/G | G/A | A/A | G/G or G/A | A/A |
| With early withdrawal | 53 | 38 | 8 | 91 | 8 |
| comprising delusions and hallucinations (99) | (53.5%) | (38.4%) | (8.1%) | (91.9%) | (8.1%) |
| | | | | | |
| With prolonged delusions and | 32 | 32 | 1 | 64 | 1 |
| hallucinations (65) | (492%) | (492%) | (1.5%) | (98.5%) | (1.5%) |
| | | | χ² = 4.283 | | χ² = 3.238 |
| | | | P = 0.1175 | | P = 0.0720 |

The results from Examples 3-6 above proved that the determination of the genotype of a *POMC* polymorphism, G-1695A, also allows easy prediction of drug sensitivity relative to vulnerability to drug addiction.

### INDUSTRIAL APPLICABILITLY

The present invention provides a genetic polymorphism in the 5' flanking region of the *POMC* gene that allows evaluation of the difference in drug sensitivity among individuals and a method for evaluating drug sensitivity by using the genetic polymorphism. Use of this evaluation method allows one to easily determine an optimal dosage to be prescribed and an optimal schedule and the like associated with a narcotic analgesic, such as morphine, which is extremely beneficial for personalized pain therapy and treatment of drug dependence.

### SEQUENCE LISTING FREE TEXT

SEQ ID NO:7: Synthetic DNA
SEQ ID NO:8: Synthetic DNA
SEQ ID NO:9: Synthetic DNA
SEQ ID NO:10: Synthetic DNA
SEQ ID NO:11: Synthetic DNA
SEQ ID NO:12: Synthetic DNA
SEQ ID NO:13: Synthetic DNA
SEQ ID NO:14: Synthetic DNA
SEQ ID NO:15: Synthetic DNA
SEQ ID NO:16: Synthetic DNA

## Claims

1. A method for evaluating drug sensitivity, comprising associating the rs3754860 genetic polymorphism in the 5' flanking region *of POMC* gene with an individual drug sensitivity, wherein the drug is an opioid receptor function-modifying agent.

2. A method according to claim 1, wherein the opioid receptor function-modifying agent is at least one selected from the group consisting of methamphetamine, methylenedioxymethamphetamine, amphetamine, dextroamphetamine, dopamine, cocaine, heroin, morphine, hydromorphone, oxymorphone, DAMGO, codeine, dihydrocodeine, methadone, oxycodone, hydrocodone, fentanyl, carfentanil, tramadol, meperidine, dextropropoxyphene, dextromoramide, deltorphin, DPDPE, DADLS, DSTLE, DAMEA, U50488H, ketocyclazocine, enadoline, bremazocine, levorphanol, etorphine, dihydroetorphine, buprenorphine, nalorphine, nalbuphine, pentazocine, levallorphan, butorphanol, naltrexone, alvimopan, naltrindole, norbinaltorphimine (nor-BNI), Mr2266, naloxone, methanol, ethanol, propanol, butanol, pentanol, and diethyl ether.

3. A method for determining the type, the number of doses, and/or the dosage of an opioid receptor function-modifying agent to be administered to an individual, comprising using the results from the evaluation by the method according to claim 1 or 2 as an index.

4. A method for predicting a side effect of an opioid receptor function-modifying agent to be administered to an individual, comprising using the results from the evaluation by the method according to claim 1 or 2 as an index.

5. Use of an oligonucleotide comprising a nucleotide sequence of at least 10 nucleotides from SEQ ID NO: 1 or 2, including the 5 1 st nucleotide of SEQ ID NO: 1 if the nucleotide sequence is derived from SEQ ID NO: 1 or the 51^{st} nucleotide of SEQ ID NO: 2 if the nucleotide sequence is derived form SEQ ID NO: 2, for evaluating drug sensitivity to an opioid receptor function-modifying agent.

6. Use according to claim 5, wherein the oligonucleotide spans a length of 10 to 45 nucleotides.

7. Use of an oligonucleotide comprising a nucleotide sequence represented by SEQ ID NO:1 or 2 for evaluating drug sensitivity to an opioid receptor function-modifying agent.

8. Use according to any one of claims 5 to 7, wherein a microarray is used comprising an oligonucleotide as defined in any one of claims 5 to 7 immobilized on a support.

9. A kit for evaluating drug sensitivity to an opioid receptor function-modifying agent, comprising:
(a) the oligonucleotide according to any one of claims 5 to 7 and/or the microarray according to claim 8; and
(b) an opioid receptor function-modifying agent.

## Patentansprüche

1. Verfahren zur Bewertung der Arzneimittelsensibilität, das das Assoziieren des rs3754860 genetischen Polymorphismuses in der 5'-flankierenden Region des POMC-Gens mit einer individuellen Arzneimittelsensibilität umfasst, wobei das Arzneimittel ein die Opioid-Rezeptorfunktion modifizierendes Agens ist.

2. Verfahren gemäss Anspruch 1, wobei das die Opioid-Rezeptorfunktion modifizierende Agens mindestens eines, ausgewählt aus der Gruppe bestehend aus Methamphetamin, Methylendioxymethamphetamin, Amphetamin, Dextroamphetamin, Dopamin, Kokain, Heroin, Morphin, Hydromorphon, Oxymorphon, DAMGO, Codein, Dihydrocodein, Methadon, Oxycodon, Hydrocodon, Fentanyl, Carfentanil, Tramadol, Meperidin, Dextropropoxyphen, Dextromoramid, Deltorphin, DPDPE, DADLS, DSTLE, DAMEA, U50488H, Ketocyclazocin, Enadolin, Bremazocin, Levorphanol, Etorphin, Dihydroetorphin, Buprenorphin, Nalorphin, Nalbuphin, Pentazocin, Levallorphan, Butorphanol, Naltrexon, Alvimopan, Naltrindol, Norbinaltorphimin (nor-BNI), Mr2266, Naloxon, Methanol, Ethanol, Propanol, Butanol, Pentanol und Diethylether, ist.

3. Verfahren zur Bestimmung der Art, der Anzahl der Dosen und/oder der Dosierung eines die Opioid-Rezeptorfunktion modifizierenden Agens, das einem Individuum verabreicht werden soll, umfassend die Verwendung der Bewertungsergebnisse aus dem Verfahren gemäss Anspruch 1 oder 2 als Index.

4. Verfahren zur Vorhersage eines Nebeneffektes eines die Opioid-Rezeptorfunktion modifizierenden Agens, das einem Individuum verabreicht werden soll, umfassend die Verwendung der Bewertungsergebnisse aus dem Verfahren gemäss Anspruch 1 oder 2 als Index.

5. Verwendung eines Oligonukleotids, umfassend eine Nukleotidsequenz mit mindestens 10 Nukleotiden von SEQ ID NO: 1 oder 2, einschliesslich dem 51. Nukleotid von SEQ ID NO: 1, wenn die Nukleotidsequenz von SEQ ID NO: 1 abgeleitet ist, oder dem 51. Nukleotid von SEQ ID NO: 2, wenn die Nukleotidsequenz von SEQ ID NO: 2 abgeleitet ist, zur Bewertung der Arzneimittelsensibilität eines die Opioid-Rezeptorfunktion modifizierenden Agens.

6. Verwendung gemäss Anspruch 5, wobei das Oligonukleotid eine Länge von 10 bis 45 Nukleotiden hat.

7. Verwendung eines Oligonukleotids, umfassend eine durch SEQ ID NO: 1 oder 2 dargestellte Nukleotidsequenz, zur Bewertung der Arzneimittelsensibilität eines die Opioid-Rezeptorfunktion modifizierenden Agens.

8. Verwendung gemäss irgendeinem der Ansprüche 5 bis 7, wobei ein Microarray verwendet wird, der ein Oligonukleotid wie in irgendeinem der Ansprüche 5 bis 7 definiert, immobilisiert auf einem Träger, umfasst.

9. Kit zur Bewertung der Arzneimittelsensibilität eines die Opioid-Rezeptorfunktion modifizierenden Agens, umfassend:
(a) das Oligonukleotid gemäss irgendeinem der Ansprüche 5 bis 7 und/oder den Microarray gemäss Anspruch 8; und
(b) ein die Opioid-Rezeptorfunktion modifizierendes Agens.

## Revendications

1. Procédé pour évaluer une sensibilité à un médicament, qui consiste à associer le polymorphisme génétique rs3754860 dans la région flanquante 5' du gène *POMC* à une sensibilité individuelle à un médicament, où le médicament est un agent modifiant la fonction d'un récepteur opioïde.

2. Procédé selon la revendication 1, dans lequel l'agent modifiant la fonction d'un récepteur opioïde est au moins un agent sélectionné dans le groupe consistant en la méthamphétamine, la méthylènedioxyméthamphétamine, l'amphétamine, la dextroamphétamine, la dopamine, la cocaïne, l'héroïne, la morphine, l'hydromorphone, l'oxymorphone, DAMGO, la codéine, la dihydrocodéine, la méthadone, l'oxycodone, l'hydrocodone, le fentanyl, le carfentanil, le tramadol, la mépéridine, le dextropropoxyphène, le dextromoramide, la deltorphine, DPDPE, DADLS, DSTLE, DAMEA, U50488H, la kétocyclazocine, l'énadoline, la brémazocine, le lévorphanol, l'étorphine, la dihydroétorphine, la buprénorphine, la nalorphine, la nalbuphine, la pentazocine, le levallorphan, le butorphanol, la naltrexone, l'alvimopan, la naltrindole, la norbinaltorphimine (nor-BNI), Mr2266, la naloxone, le méthanol, l'éthanol, le propanol, le butanol, le pentanol, et l'éther diéthylique.

3. Procédé pour déterminer le type, le nombre de doses, et/ou la forme posologique d'un agent modifiant la fonction d'un récepteur opioïde devant être administré à un individu, qui consiste à utiliser les résultats de l'évaluation par le procédé selon la revendication 1 ou 2 en tant qu'index.

4. Procédé pour prédire un effet secondaire d'un agent modifiant la fonction d'un récepteur opioïde devant être administré à un individu, qui consiste à utiliser les résultats de l'évaluation par le procédé selon la revendication 1 ou 2 en tant qu'index.

5. Utilisation d'un oligonucléotide comprenant une séquence de nucléotides d'au moins 10 nucléotides parmi SEQ ID NO: 1 ou 2, comprenant le 51^{e} nucléotide de SEQ ID NO: 1 si la séquence de nucléotides est dérivée de SEQ ID N0: 1 ou le 51^{e} nucléotide de SEQ ID NO: 2 si la séquence de nucléotides est dérivée de SEQ ID NO: 2, afin d'évaluer une sensibilité à un médicament par rapport à un agent modifiant la fonction d'un récepteur opioïde.

6. Utilisation selon la revendication 5, dans laquelle l'oligonucléotide couvre une longueur de 10 à 45 nucléotides.

7. Utilisation d'un oligonucléotide comprenant une séquence de nucléotides représentée par SEQ ID NO: 1 ou 2 afin d'évaluer une sensibilité à un médicament par rapport à un agent modifiant la fonction d'un récepteur opioïde.

8. Utilisation selon l'une quelconque des revendications 5 à 7, dans laquelle un microréseau est utilisé comprenant un oligonucléotide tel que défini dans l'une quelconque des revendications 5 à 7 immobilisé sur un support.

9. Kit pour évaluer une sensibilité à un médicament par rapport à un agent modifiant la fonction d'un récepteur opioïde, comprenant :
(a) l'oligonucléotide selon l'une quelconque des revendications 5 à 7 et/ou le microréseau selon la revendication 8 ; et
(b) un agent modifiant la fonction d'un récepteur opioïde.


### SEQUENCE LISTING

<110> TOKYO METROPOLITAN ORGANIZATION FOR MEDICAL RESEARCH
<120> A METHOD OF EVALUATING DRUG SENSITIVITY WITH ANALYSES OF POMC GENE
<130> PCT08-0028
<150> JP 2007-114968
   <151> 2007-04-25
<160> 16
<170> PatentIn version 3.4
<210> 1
   <211> 101
   <212> DNA
   <213> Homo Sapiens
<400> 1
   accaaaaccc aaaattagct gggcatggtg gcatgcatct gtggttccag rtactcagga 60
   ggctgaagca tgaggatcac ttgagcccag gaggtggagg c 101
<210> 2
   <211> 101
   <212> DNA
   <213> Homo Sapiens
<400> 2
   gcctccacct cctgggctca agtgatcctc atgcttcagc ctcctgagta yctggaacca 60
   cagatgcatg ccaccatgcc cagctaattt tgggttttgg t 101
<210> 3
   <211> 101
   <212> DNA
   <213> Homo Sapiens
<400> 3
   ccgcgaagct gcaggcgctg tctccaggga gccggcggcc tcctctcccc saggggctcg 60
   cggcggtccg gaggctccga gagcttgcta ggaggtcttg g 101
<210> 4
   <211> 101
   <212> DNA
   <213> Homo Sapiens
<400> 4
   ccaagacctc ctagcaagct ctcggagcct ccggaccgcc gcgagcccct sggggagagg 60
   aggccgccgg ctccctggag acagcgcctg cagcttcgcg g 101
<210> 5
   <211> 101
   <212> DNA
   <213> Homo Sapiens
<400> 5
   gccccagggc taccctcccc caggaggtcg accccaaagc cccttgctct yccctgccct 60
   gctgccgcct cccagcctgg ggggtcgtgg cagataatca g 101
<210> 6
   <211> 101
   <212> DNA
   <213> Homo Sapiens
<400> 6
   ctgattatct gccacgaccc cccaggctgg gaggcggcag cagggcaggg ragagcaagg 60
   ggctttgggg tcgacctcct gggggagggt agccctgggg c 101
<210> 7
   <211> 21
   <212> DNA
   <213> Artificial
<220>
   <223> synthetic DNA
<400> 7
   ccagtgattc taaatgcagt t 21
<210> 8
   <211> 21
   <212> DNA
   <213> Artificial
<220>
   <223> synthetic DNA
<400> 8
   gtaacttcag gaggctgctg g 21
<210> 9
   <211> 21
   <212> DNA
   <213> Artificial
<220>
   <223> synthetic DNA
<400> 9
   ccagtgattc taaatgcagt t 21
<210> 10
   <211> 23
   <212> DNA
   <213> Artificial
<220>
   <223> synthetic DNA
<400> 10
   gaattagggt ctcactctgt tgc 23
<210> 11
   <211> 21
   <212> DNA
   <213> Artificial
<220>
   <223> synthetic DNA
<400> 11
   ccagtgattc taaatgcagt t 21
<210> 12
   <211> 24
   <212> DNA
   <213> Artificial
<220>
   <223> synthetic DNA
<400> 12
   gcagatggtg gttttgcctt attt 24
<210> 13
   <211> 20
   <212> DNA
   <213> Artificial
<220>
   <223> synthetic DNA
<400> 13
   agcctcccga gacaggtaag 20
<210> 14
   <211> 20
   <212> DNA
   <213> Artificial
<220>
   <223> synthetic DNA
<400> 14
   cctgttgtcg ggagtttgag 20
<210> 15
   <211> 18
   <212> DNA
   <213> Artificial
<220>
   <223> synthetic DNA
<400> 15
   cccaaggaca agcgctac 18
<210> 16
   <211> 20
   <212> DNA
   <213> Artificial
<220>
   <223> synthetic DNA
<400> 16
   ccatgctgct gttatttgac 20